# FASCICULE DE BREVET EUROPEEN

(11) **EP 3 522 895 B1**
(45) Date de publication et mention de la délivrance du brevet: **01.11.2023**
(21) Numéro de dépôt: 17777921.2
(22) Date de dépôt: 05.10.2017
(51) Int. Cl.: A61K 31/7028, A61P 17/00, A61P 17/02, A61K 8/60, A61Q 19/00, A61K 9/00

(54) **UTILISATION DE 6-O-(C8-C20 ALKYL ESTER) DE 1-O-(C1-C6 ALKYL)-SS-D-GLUCOSIDE COMME AGENT DE RÉGÉNÉRATION ET/OU DE RÉPARATION CELLULAIRE DE L'ÉPIDERME**
VERWENDUNG VON 6-O-(C8-C20 ALKYL ESTER) VON 1-O-(C1-C6 ALKYL)-SS-D-GLUCOSIDE ALS MITTEL ZUR REGENERATION UND/ODER ZELLERNEUERUNG DER EPIDERMIS
USE OF 6-O-(C8-C20 ALKYL ESTER) OF 1-O-(C1-C6 ALKYL)-SS-D-GLUCOSIDE AS AGENT FOR REGENERATION AND/OR CELLULAR REPARATION OF THE EPIDERMIS

(30) Priorité: 05.10.2016 FR 1659616
(43) Date de publication de la demande: 14.08.2019
(73) Titulaire: Pierre Fabre Dermo-Cosmétique, 81500 Lavaur (FR)
(72) Inventeur: DAUNES MARION, Sylvie, 31000 Toulouse (FR); GALLIANO, Marie Florence, 31700 Blagnac (FR); HERNANDEZ-PIGEON, Hélène, 31270 Cugnaux (FR); POIGNY, Stéphane, 31600 Saubens (FR)
(74) Mandataire: Regimbeau
(86) Numéro de dépôt international: PCT/EP2017/075406
(87) Numéro de publication internationale: WO 2018/065545

(56) Documents cités:
- WO-A1-95/07967
- WO-A1-2010/093065
- FR-A1- 2 896 691

## Description

La présente invention concerne l'utilisation de 6-O-(C8-C20 alkyl ester) de 1-O-(C1-C6 alkyl)- β-D-glucoside et notamment du 6-O-lauroyl de 1-O-n-butyl-β-D-glucoside comme agent protecteur de la peau et/ou des muqueuses. Plus particulièrement, elle concerne une composition cosmétique, dermatologique et/ou pharmaceutique, destinée à un usage topique, utilisable pour un soin et/ou un traitement de la peau.

Ces dérivés glucosidiques sont utiles comme actifs agissant sur l'hydratation de la peau, le renforcement de la fonction barrière, le maintien de l'homéostasie épidermique, la résistance à différents stress, la réparation cellulaire et la régénération tissulaire.

FR 2896691 divulgue l'utilisation d'esters d'alkylglucoside, et en particulier l'ester de laurate de butylglucoside comme agent antimicrobien.

WO 95/07967 divulgue des composés du type esters d'alkylglucoside et leur utilisation pour enlever des silicones d'une surface.

WO 2010/093065 divulgue une composition pour la régénération de la peau et pour faciliter la cicatrisation, comprenant comme agent actif régénérant l'ascorbyl glucoside.

### Homéostasie de l'épiderme

L'épiderme joue un rôle de protection majeur en tant que barrière mécanique et chimique pour le corps. Il permet d'assurer qu'une fonction barrière cutanée imperméable soit maintenue. Ce sont les cornéocytes qui sont les kératinocytes de la couche cornée, conjointement avec une matrice lipidique qui assurent cette fonction en grande partie. Néanmoins, les couches les plus profondes contribuent également à construire les éléments inhérents à cette fonction. La capacité de différenciation des kératinocytes épidermiques fait en sorte de construire une barrière ayant la fonction de perméabilité sélective (Elias and Choi, Exp. Dermatol. 14(10), p19-26, 2005). La différenciation des kératinocytes est régulée dans l'espace et dans le temps, à partir des couches les plus profondes de la peau, la membrane basale étant la moins différenciée, vers la couche cornée ou stratum cornea, étape finale de différenciation des kératinocytes en cornéocytes (Houben et al., Skin Pharmacol. Physiol. 20(3), p122-132, 2007). D'un point de vue cellulaire et moléculaire, la formation de filaments de kératine, la transformation des kératinocytes en cornéocytes ou « kératinisation », et la formation d'un ciment lipidique intercellulaire de structure lamellaire sont principalement observées, assurant l'imperméabilité et la fonction de barrière cutanée.

En termes de protéines, la différenciation épidermique se concentre principalement sur le développement de protéines de structure que sont les kératines, et qui contribuent à l'intégrité architecturale de l'épiderme. Leur expression varie en fonction du niveau de maturation des kératinocytes. La kératine alcaline 1 et la kératine acide 10 sont des marqueurs précoces de la différenciation des kératinocytes, présents dans la membrane basale de l'épiderme. L'expression d'autres marqueurs dans ce processus biologique, qui a lieu plus tard, peut être suivie de la même manière que pour l'enveloppe cornée, tels que l'involucrine, conjointement avec certaines enzymes principales amenant les protéines de structure à former des ponts entre elles et avec les lipides des kératinocytes et les transglutaminases, telles que la TGM1 ou 3 (Houben et al., Skin Pharmacol. Physiol. 20(3), p122-132, 2007).

La matrice fibreuse présente dans les cornéocytes est formée lors de la transition entre les kératinocytes granulaires et les cornéocytes. La loricrine est une protéine de structure contenant des résidus glutamine et lysine qui permettent l'adhérence à d'autres protéines dans l'enveloppe cornée. Les molécules de filaggrine basique produites à partir de leur précurseur, à savoir la profilaggrine, stockée dans les granules de kératohyaline, se combinent aux filaments de cytokératine, pour ensuite pouvoir s'agréger. La filagrine, dégradée par la caspase 14, représente également la source principale de plusieurs constituants majeurs du facteur naturel d'hydratation dans la couche cornée. D'autres marqueurs sont spécifiques des kératinocytes différenciés. Les kallicréines, telles que la KLK5 et la KLK7, présentent une activité similaire à celle de la chymotrypsine et jouent un rôle dans la protéolyse de structures cohésives intercellulaires qui précèdent la desquamation, à savoir l'élimination de la couche la plus externe de l'épiderme.

Parallèlement, la synthèse et le transport des lipides des kératinocytes forment la base du ciment lipidique intercornéocytes, essentiel à la barrière cutanée, dont la formation représente la phase finale dans la différenciation épidermique terminale. Cette matrice lipidique extra-cellulaire constitue la barrière principale pour le transport transcutané des fluides et des électrolytes (Feingold, J. Lipid Res. 48, p2529-2530, 2007). Ainsi, un certain nombre d'enzymes et de transporteurs lipidiques, voient régulée à la hausse l'expression de leurs kératinocytes conjointement avec la différenciation. Le ciment résulte de l'équilibre entre trois espèces lipidiques, à savoir le cholestérol, les acides gras libres et les céramides. Ces lipides proviennent de glucosylcéramides, de la sphingomyéline, du cholestérol et de phospholipides produits dans la couche épineuse ou *stratum spinosum* et la couche granuleuse ou *stratum granulosum.* Ils sont transportés par les corps lamellaires, qui sont des organites sécrétoires qui fusionnent avec la couche granuleuse et la couche cornée. En plus de ces précurseurs lipidiques, les corps lamellaires contiennent de nombreuses enzymes, y compris des lipidases telles qu'une sphingomyélinase acide, une bêta-glucocérébrosidase ou des phospholipases A2, conjointement avec des lipases acides et neutres. Délivrées en même temps aux espaces extracellulaires que les précurseurs lipidiques, ces enzymes convertissent respectivement la sphingomyéline en céramide, les glucocérébrosides en céramide et les phospholipides en acides gras libres et en glycérol. La SULT2B1 est une cholestérol sulfotransférase exprimée dans les kératinocytes différenciés et est impliquée dans la synthèse du sulfate de cholestérol. Une étude récente a également révélé que le sulfate de cholestérol induit l'expression de la filaggrine par une expression accrue de RORα (Hanyu et al., Biochem. Biophys. Res. Commun. 428(1), p99-104, 2012).

Les céramides épidermiques jouent un rôle spécifique majeur et représentent un marqueur essentiel du niveau de fonctionnalité de la barrière cutanée. Les enzymes jouant un rôle dans la production de céramides de la peau, voient leur expression et leur activité augmenter spécifiquement, lorsque la fonction de barrière cutanée est altérée, et conjointement avec le niveau de différenciation épidermique (Feingold, J. Lipid Res. 48, p2529-2530, 2007). Ceci est le cas spécifique d'une aSmase et de la β-glucocéramidase, impliquées dans le métabolisme extracellulaire des céramides de la peau. L'UGCG (UDP-Glucose Céramide) Glucosyltransférase) est également impliquée dans la synthèse de glucosylcéramides. L'UGCG catalyse la première étape de glycosylation dans la biosynthèse des glycosphingolipides et est nécessaire pour l'arrangement régulier des lipides et des protéines dans les corps lamellaires et pour le maintien de la barrière épidermique (Jennemann et al., J. Biol. Chem. 282(5), p3083-3094, 2007). La DEGS2 agit à la fois en tant que sphingolipides C4-hydroxylase et en tant que delta-4 désaturase, son activité dihydrocéramide hydroxylase étant en compétition avec la production de phytocéramides de la peau chez l'homme (Mizutani et al., FEBS Lett. 563(1-3), p93-97, 2004).

La FABP-E (FABP5), protéine de liaison aux acides gras épidermique, est un transporteur lipidique. FABP-E joue un rôle important dans la différenciation des kératinocytes (Dallaglio, et al., Exp Dermatol. 22(4), p255-261, 2013).

L'une des fonctions de l'eau dans la couche cornée est d'activer des réactions d'hydrolyse enzymatique nécessaires à la souplesse de la peau et à une desquamation normale (Rawlings and Matts, J. Invest. Dermatol., 124(6), p1099-1110, 2005). Si la teneur en eau dans la couche cornée chute au-dessous d'un niveau critique, les réactions enzymatiques sont perturbées, menant une adhérence des cornéocytes et à l'accumulation des cellules à la surface de la peau. Ceci crée une sécheresse visible et un prurit, la peau pèle et s'exfolie.

L'hydratation de la peau repose sur deux points, l'apport en eau transépidermique à partir de la circulation sanguine cutanée et la rétention d'eau épidermique qui met en jeu la fonction de barrière cutanée. Toutefois, la barrière à la perte hydrique n'est pas infaillible. Un échange normal d'eau entre les environnements extérieur et intérieur à travers la couche cornée est connu sous le nom de TEWL (Transepidermal Water Loss ou Perte d'eau transépidermique) et est inhérent à une perte insensible d'eau (IWL ou Insensible Water Loss).

### La cicatrisation

La cicatrisation, la réparation cellulaire et la régénération de l'épiderme associées, est classiquement découpée en quatre phases : l'hémostase, l'inflammation, la réparation et le remodelage (Reinke and Sorg, Eur. Surg. Res., 49, p35-43, 2012). Deux à dix jours après une blessure, on assiste à une forte prolifération et migration cellulaire afin de restaurer rapidement le tissu et sa vascularisation. Durant cette phase, la réépidermisation ou ré-épithélialisation est le processus qui recouvre une plaie par régénération des kératinocytes de l'épiderme. Ceci permet de restaurer la fonction barrière protectrice contre l'environnement extérieur et ainsi de réduire la morbidité et la mortalité à la suite d'une blessure.

La réépidermisation se compose de trois phénomènes, se déroulant en parallèle mais décalés dans le temps, dans l'épiderme en régénération :
- la migration de kératinocytes
- la prolifération des kératinocytes
- la maturation du néoépiderme.

Suite à la rupture de l'épiderme, les kératinocytes deviennent activés en subissant des modifications au niveau de leur cytosquelette. Les kératinocytes en migration prennent la forme d'une langue de ré-épithélialisation, ces kératinocytes en migration ne suivent pas le schéma de différenciation habituel mais restent dans un stade indifférencié exprimant des kératines de la couche basale (5 et 14) mais peu de protéines caractéristiques de la différenciation. En arrière du front de migration les kératinocytes entrent en prolifération. Cette prolifération limitée à la couche basale est induite par une combinaison de facteurs de croissance, dont l'EGF, le TGF-α et GM-CSF. Une fois la plaie complètement épithélialisée, les kératinocytes intègrent leur programme de différenciation terminale et commencent leur stratification. Les tissus nécrosés et le caillot sanguin sont repoussés par les cellules du néoépiderme en hyperprolifération dont les couches superficielles desquament.

Ainsi, la migration des kératinocytes est un processus essentiel dès les premiers jours après une blessure pour permettre la régénération de l'épiderme, en particulier du néoépiderme, ainsi qu'une réparation cellulaire.

La migration des kératinocytes peut être évaluée *in vitro* à partir de cultures de kératinocytes primaires humains grâce à des techniques opératoires standardisées telles que celles développée dans le test Oris^{™} Cell migration assay (Platypus Technologies-TEBU).

L'objet de la présente invention concerne au moins un composé 6-O-(C8-C20 alkyl ester) de 1-O-(C1-C6 alkyl)-β-D-glucoside pour son utilisation comme agent de régénération de l'épiderme et/ou de réparation cellulaire de l'épiderme. De façon préférée, l'objet de l'invention concerne le 6-O-lauroyl de 1-On-butyl-P-D-glucoside pour son utilisation comme agent régénération de l'épiderme et/ou de réparation cellulaire de l'épiderme.

La demanderesse a montré qu'un composé 6-O-(C8-C20 alkyl ester) de 1-O-(C1-C6 alkyl)-β-D-glucoside et plus particulièrement le 6-O-lauroyl de 1-O-n-butyl-β-D-glucoside permet une une hydratation de la peau, un renforcement de la fonction de barrière de l'épiderme, un maintien de l'homéostasie épidermique, un résistance à différents stress. Ce faisant, le composé selon l'invention contribue à la réparation cellulaire de l'épiderme et/ou à la régénération de l'épiderme ou encore à la cicatrisation. Cet effet a pu être démontré par une induction d'expression d'ARNm de divers gènes impliqués dans la différenciation des kératinocytes et l'hydratation, mais aussi au travers de la stimulation de la migration des kératinocytes.

Par hydratation de la peau, on entend l'amélioration ou le maintien de l'équilibre en eau de la peau.

Par l'amélioration de toute forme de sécheresse cutanée on entend toute amélioration de l'hydratation de l'épiderme notamment caractérisée par un manque d'eau dans la couche cornée, un film hydrolipidique situé à la surface trop fin et qui ne protège plus la peau, le manque de sébum.

L'objet de la présente invention concerne également une composition dermatologique, cosmétique ou pharmaceutique, comprenant, ou consistant en, à titre de principe actif, au moins un composé 6-O-(C8-C20 alkyl ester) de 1-O-(C1-C6 alkyl)-β-D-glucoside et au moins un excipient acceptable, pour son utilisation dans la régénération de l'épiderme et/ou la réparation cellulaire de l'épiderme.

De manière préférée, l'utilisation est une utilisation topique.

La composition dermatologique, cosmétique ou pharmaceutique, qui comprend à titre de principe actif au moins un composé 6-O-(C8-C20 alkyl ester) de 1-O-(C1-C6 alkyl)-β-D-glucoside et au moins un excipient acceptable, est également utile pour favoriser la réparation cellulaire de l'épiderme et/ou favoriser la régénération de l'épiderme, voire encore favoriser la cicatrisation.

D'une façon avantageuse, la composition selon l'invention est destinée à une application topique sur la peau et les phanères, le cuir chevelu et les muqueuses.

Dans un mode particulier de l'invention, le 6-O-(C8-C20 alkyl ester) de 1-O-(C1-C6 alkyl)-β-D-glucoside, plus particulièrement le 6-O-lauroyl de 1-O-n-butyl-β-D-glucoside, est préféré à titre de principe actif dans la composition.

De manière plus préférée le 6-O-(C8-C20 alkyl ester) de 1-O-(C1-C6 alkyl)-β-D-glucoside, plus particulièrement le 6-O-lauroyl de 1-O-n-butyl-β-D-glucoside, est le seul principe actif de la composition ayant un effet sur la réparation cellulaire de l'épiderme et/ou la régénération de l'épiderme, voire sur la cicatrisation.

Selon l'invention, la composition dermatologique ou cosmétique, pour son utilisation selon l'invention, peut comprendre de 0,01 à 10%, de préférence de 0,1% à 5%, et plus particulièrement 0,1 à 3% et encore plus particulièrement de 0,1 à 2% en poids 6-O-lauroyl de 1-O-n-butyl-β-D-glucoside par rapport au poids total de la composition.

Dans un mode de réalisation préféré, la composition pour son utilisation selon l'invention se présentera préférentiellement sous la forme d'un produit de soin et/ou de maquillage de la peau du corps ou du visage, des lèvres, des cils, des sourcils, des cheveux, du cuir chevelu, de ongles ou des muqueuses, d'un produit solaire ou autobronzant, d'un produit capillaire notamment de coloration, de conditionnement et/ou de soin des cheveux.

L'administration topique de la composition dermatologique ou cosmétique peut être accomplie par application d'une solution, d'une suspension, d'un gel aqueux, d'une lotion, d'un sérum, d'un lait, d'une pommade, d'un onguent, d'une crème, d'un collyre, ou un autre véhicule utilisé pour une application topique, bien connu de l'homme du métier, notamment sous forme hydro alcoolique, d'une émulsion huile-dans-eau ou eau-dans-huile ou multiple, d'un gel huileux, ou d'un produit anhydre liquide, pâteux ou solide ou sous forme de dispersion en présence de sphérules. Ces compositions sont préparées selon les méthodes usuelles. L'un des moyens possibles est l'administration de la composition dermatologique ou cosmétique par un spray aérosol permettant de vaporiser un liquide en fines gouttelettes pour une distribution sur toute la surface nécessaire ou au contraire de limiter avec précision une zone particulière à viser ou encore sous forme solide, sous forme de stick. Un autre exemple de réalisation est le patch ou timbre qui fournit une libération continue de la composition topique. Le patch peut avoir un réservoir et une membrane poreuse ou une matrice solide qui sont bien connus de l'homme de l'art.

Les huiles utilisables dans l'invention sont celles généralement utilisées dans les domaines concernés. Elles peuvent être végétales, minérales ou synthétiques, éventuellement siliconées et/ou fluorées.

Les compositions topiques pour un utilisation selon l'invention peuvent contenir également des adjuvants hydrophiles ou lipophiles come des gélifiants, des conservateurs, des opacifiants, des émulsionnants, de coémulsionnants, des neutralisants, des parfums, et leurs solubilisants ou peptisants, des colorants, des pigments, des antiseptiques, des antioxydants, des sels minéraux, des épaississants, des modificateurs de pH, des agents absorbant les rayons ultraviolets, des vitamines ou tout autre excipient acceptable en cosmétologie et en dermatologie que l'homme du métier connaît bien.

La composition pour son utilisation selon l'invention peut en outre contenir un autre agent actif par voie topique, ou un mélange de tels agents actifs.

Par tensioactif, on entend de manière classique, toute molécule amphiphile capable d'agir sur la tension interfaciale d'un milieu dispersé. La composition selon l'invention est alors exempte de tel agent tensioactif susceptible de solubiliser le film hydrolipidique de la peau. Avantageusement, elle peut être également dépourvue de conservateurs, susceptibles d'être responsables d'intolérances cutanées, notamment de tout ammonium quaternaire, éthanol, phénols, amidines, dérivés d'isothiazolone, esters parahydroxybenzoïques (connus sous le nom de parabens), etc.

Les compositions décrites peuvent être appliquées sur la surface à traiter de la peau du patient. La fréquence d'application dépendra des circonstances et de la personne. Par exemple, les compositions peuvent être appliquées quotidiennement, deux fois par jour ou même plus fréquemment.

Un glucoside implique un sucre (typiquement un ose) lié à une substance non glucidique par une liaison de type O-. En particulier on distingue les alpha (ou α) glucosides et les beta (ou β) glucosides. Les beta-glucosides ont une liaison osidique de forme beta qui est une liaison chimique covalente entre le groupe groupement réducteur (hydroxyle) de la fonction alcool du carbone hémiacétalique d'un ose (carbone anomère, numéro 1 chez les aldoses tel que le glucose) avec le groupement acide (hydrogène libre) d'une autre molécule.

Dans le cas présent on parle d'un alkyl D glucoside de forme beta. Il s'agit d'un glucoside impliquant le D glucose dont la fonction alcool du carbone 1 hemiacétal en conformation beta a été condensée avec une molécule à chaîne alkyl disposant d'un hydroxyl, ici un alcool à chaîne grasse qui est donc en position 1.

Pour ce qui est de la moitie alkyl ester de la molécule, il s'agit de l'estérification par un composé alkyl à résidu carboxylique de l'alcool secondaire du glucose, ie en position 6.

Les termes C8-C20 alkyl et C1-C6 alkyl font référence à des résidus hydrocarbonés linéaires ou ramifiés présentant entre 8 et 20 atomes de carbone et entre 1 et 6 atomes de carbone, respectivement.

La présente description divulgue aussi un procédé de préparation stéréo-sélective de 6-O-(C8-C20 alkyl ester) de 1-O-(C1-C6 alkyl)-β-D-glucoside , et notamment du 6-O-lauroyl de 1-O-n-butyl-P-D-glucoside. Ce procédé implique la mise en oeuvre des étapes successives suivantes :
a) acétalisation du D-glucose par un alcanol en C₁-C₆ en présence d'une enzyme hydrolase pour former le 1-O-(C1-C6 alkyl)-β-D-glucoside correspondant ;
b) estérification du 1-O-(C1-C6 alkyl)-β-D-glucoside obtenu à l'issue de l'étape a) par un acide gras en C₈-C₂₀ en présence d'une enzyme lipase pour former ledit 6-O-(C8-C20 alkyl ester) de 1-O-(C1-C6 alkyl)-β-D-glucoside.

Le β-LBG obtenu par la mise en oeuvre du procédé décrit ci-dessus présente des propriétés dermo-cosmétiques améliorées de façon inattendue en raison de leur forme de pureté spécifique.

Les exemples suivants illustrent la présente invention.

### Exemple 1 : évaluation du 6-O-lauroyl de 1-O-n-butyl-β-D-glucoside sur la modulation de gènes impliqués dans la différenciation des kératinocytes et l'hydratation.

Des kératinocytes normaux humains ont été incubés pendant 48h avec du 6-O-lauroyl de 1-O-n-butyl-β-D-glucoside. Ses effets ont été évalués grâce à la technique de RT-qPCR avec l'analyse de 6 gènes cibles, choisis pour leur importance dans la différenciation des kératinocytes et l'hydratation.

Les résultats de cette étude sont résumés dans le tableau 1 ci-dessous.

| Gènes | | CaCl2 | | 6-O-lauroyl de 1-O-n-butyl-β-D-glucoside | |
|---|---|---|---|---|---|
| | | 1,5mM | | 20 µM | |
| | | Moyenne (% Témoin) | SD | Moyenne (% Témoin) | SD |
| Différenciation lipidique | SULT2B1 | 1169 | 25 | 549 | 122 |
| | FABP5 | 501 | 179 | 259 | 1 |
| | DEG S2 | 217 | 125 | 277 | 16 |
| Différenciation protéique | KLK7 | 994 | 220 | 253 | 43 |
| | TGM1 | 910 | 89 | 536 | 97 |
| | CASP14 | 127 | 50 | 441 | 25 |

| | | | | | |
|---|---|---|---|---|---|
| SULT2B1 : Sulfotransferase family, cytosolic, 2B, member 1; FABP5 : Fatty acid binding protein 5; DEGS2 : Degenerative spermatocyte homolog 2, lipid desaturase; KLK7 : Kallikrein-related peptidase 7; TGM1 : Transglutaminase 1; CASP14 : Caspase 14. | | | | | |

Dans les conditions d'analyse, il a été démontré après une incubation de 48h que le 6-O-lauroyl de 1-O-n-butyl-β-D-glucoside à 20µM induit une différenciation reproductible des kératinocytes. En effet, à cette concentration, le 6-O-lauroyl de 1-O-n-butyl-β-D-glucoside induit l'expression des marqueurs de la différenciation lipidique SULT2B1, FABP5 et DEGS2. Il participe ainsi à la sulfonatation du cholestérol, à la synthèse des acides gras et des céramides. Le 6-O-lauroyl de 1-O-n-butyl-β-D-glucoside induit aussi les marqueurs protéiques de la différenciation KLK7, impliquée dans la desquamation, la transglutaminase 1 (TGM1) et la caspase 14 (CASP14), impliquée dans la dégradation de la filagrine en facteur naturel d'hydratation.

Le 6-O-lauroyl de 1-O-n-butyl-β-D-glucoside joue donc un rôle très important dans la différenciation des kératinocytes et assure la fonction barrière physique protéique et lipidique. Il permet ainsi la restauration et le renforcement de la fonction barrière, la régénération de l'épiderme et la réparation cellulaire et prévient de la déshydratation cutanée.

### Exemple 2 : évaluation du 6-O-lauroyl de 1-O-n-butyl-β-D-glucoside sur la migration des kératinocytes

Le but de l'étude décrite dans cet exemple est d'évaluer les effets du 6-O-lauroyl de 1-O-n-butyl-β-D-glucoside sur la migration de de kératinocytes primaires normaux humains.

Les tests de migrations cellulaires Oris^{™} ont été utilisés pour estimer la migration cellulaire.

Des kératinocytes primaires normaux humains isolés à partir de tissus extraits lors de chirurgie esthétique, ont été ensemencés sur des plaques 96 puits et mis en culture dans du milieu KSFM supplémenté avec 25 pg/ml d'extrait pituitaire bovin (BPE, Invitrogen) et 0,2 ng/ml d'EGF (Invitrogen). Après 24h d'incubation, le milieu de culture est remplacé par du milieu KSM sans complément. Après 24h d'incubation, les stoppers Oris^{™} sont enlevés ainsi que les composés à tester ou les contrôles positifs. Ces derniers sont le TGF-β à 5 ng/ml, l'EGF à 30 ng/ml, ou le milieu KSM supplémenté avec le BPE et l'EGF. L'incubation se poursuit pour les 24h suivantes afin de permettre la migration. A la fin de l'incubation, le colorant Calcéine AM qui pénètre dans les cellules est ajouté dans les puits et est converti en fluorescence et celle-ci est quantifiée dans une zone de détection en utilisant une microplaque monochrome (Clariostar, BMG Labteck).

Le composé testé est le 6-O-lauroyl de 1-O-n-butyl-β-D-glucoside, il est solubilisé dans du DMSO à la concentration de 100 mM. Le composé est testé à 2, 10 et 20 µM. L'effet du DMSO aux concentrations testées n'a pas d'effet sur la migration des kératinocytes. Les expériences sont répétées sur 2 donneurs avec 8 puits pour chaque condition.

La quantification de la fluorescence émise dans la zone de détection est proportionnelle à la migration des kératinocytes. Des analyses statistiques (test de Mann-Whitney) permettent d'analyser l'effet du produit. Après quantification de la fluorescence, les cellules étaient rapidement fixées dans le para formaldéhyde 4% avant coloration avec l'éosine-hématoxyline et observées sous microscope.

Les résultats de cette étude sont résumés dans le tableau 2 ci-dessous :

| % Stim | Cont nég | Contrôles positifs KSFM | | | β-LBG | | |
|---|---|---|---|---|---|---|---|
| Donneurs | KSFM | EGF ¹ | TGF | EGF | 2 µM | 10µM | 20µM |
| | | BPE ² | 5ng/ml | 30ng/ml | | | |
| 1 | 0 | 170 | 266 | 35 | -30 | 41 | 104 |
| | | * | ** | | | | ** |
| 2 | 0 | 27 | 14 | 87 | 46 | 63 | 21 |
| | | | | * | | * | |

| | | | | | | | |
|---|---|---|---|---|---|---|---|
| % Stim : % de stimulation ; Cont nég : contrôle négatif ; β-LBG : 6-O-lauroyl de 1-O-n-butyl-β-D-glucoside. EGF ¹ : 0,2ng/ml ; BPE ² : 25pg/ml. *P<0,05 ; **P<0,01. | | | | | | | |

Les contrôles positifs montrent de fortes stimulations de migration (87% jusqu'à 266%) validant ces expériences.

De façon inattendue, les inventeurs montrent que le 6-O-lauroyl de 1-O-n-butyl-β-D-glucoside stimule la migration des kératinocytes et ce sur les deux donneurs testés soit à 10µM ou à 20µM, avec une plus forte stimulation obtenue à 20µM chez le premier donneur et à 10µM chez le second donneur.

En conclusion, en utilisant le test de migration cellulaire Oris^{™} sur des kératinocytes normaux humains, le 6-O-lauroyl de 1-O-n-butyl-P-D-glucoside dans des concentrations de 10 à 20 µM stimule significativement la migration de kératinocytes normaux humains et contribue donc à la régénération de l'épiderme et à la réparation cellulaire de l'épiderme.

## Revendications

1. Utilisation non-thérapeutique d'au moins un composé 6-O-(C8-C20 alkyl ester) de 1-O-(C1-C6 alkyl)-β-D-glucoside comme agent de régénération de l'épiderme et/ou de réparation cellulaire de l'épiderme.

2. Utilisation non-thérapeutique selon la revendication 1, **caractérisée en ce qu'**il s'agit du 6-O-lauroyl de 1-O-n-butyl-β-D-glucoside.

3. Composition dermatologique, **caractérisée en ce qu'**elle comprend à titre de principe actif au moins un composé 6-O-(C8-C20 alkyl ester) de 1-O-(C1-C6 alkyl)-β-D-glucoside et au moins un excipient dermatologiquement acceptable, pour son utilisation dans la régénération de l'épiderme et/ou la réparation cellulaire de l'épiderme.

4. Composition dermatologique pour son utilisation selon la revendication 3, **caractérisée en ce que** la quantité de 6-O-(C8-C20 alkyl ester) de 1-O-(C1-C6 alkyl)-β-D-glucoside varie entre 0,01% et 10% en poids par rapport au poids total de la composition.

5. Composition dermatologique pour son utilisation selon l'une des revendications 3 ou 4, **caractérisée en ce que** le principe actif est le 6-O-lauroyl de 1-O-n-butyl-β-D-glucoside.

6. Utilisation non-thérapeutique d'une composition cosmétique comprenant à titre de principe actif au moins un composé 6-O-(C8-C20 alkyl ester) de 1-O-(C1-C6 alkyl)- β-D-glucoside et au moins un excipient cosmétiquement acceptable pour la régénération de l'épiderme et/ou la réparation cellulaire de l'épiderme.

## Patentansprüche

1. Nicht-therapeutische Verwendung mindestens einer 6-O-(C8-C20 Alkylester)-Verbindung von 1-O-(C1-C6 Alkyl)-β-D-glucosid als Mittel zur Regeneration der Epidermis und/oder zur Zellreparatur der Epidermis.

2. Nicht-therapeutische Verwendung nach Anspruch 1, **dadurch gekennzeichnet, dass** es sich um 6-O-Lauroyl von 1-O-n-Butyl-β-D-glucosid handelt.

3. Dermatologische Zusammensetzung, **dadurch gekennzeichnet, dass** sie als Wirkprinzip mindestens eine 6-O-(C8-C20 Alkylester)-Verbindung von 1-O-(C1-C6 Alkyl)-β-D-glucosid und mindestens einen dermatologisch akzeptablen Hilfsstoff für ihre Verwendung zur Regeneration der Epidermis und/oder zur Zellreparatur der Epidermis umfasst.

4. Dermatologische Zusammensetzung für ihre Verwendung nach Anspruch 3, **dadurch gekennzeichnet, dass** die Menge 6-O-(C8-C20 Alkylester) von 1-O-(C1-C6 Alkyl)-β-D-glucosid zwischen 0,01 und 10 Gew.-% im Verhältnis zum Gesamtgewicht der Zusammensetzung schwankt.

5. Dermatologische Zusammensetzung für ihre Verwendung nach einem der Ansprüche 3 oder 4, **dadurch gekennzeichnet, dass** das Wirkprinzip 6-O-Lauroyl von 1-O-n-Butyl-β-D-glucosid ist.

6. Nicht-therapeutische Verwendung einer kosmetischen Zusammensetzung, die als Wirkprinzip mindestens eine 6-O-(C8-C20 Alkylester)-Verbindung von 1-O-(C1-C6 Alkyl)-β-D-glucosid und mindestens einen kosmetisch akzeptablen Hilfsstoff zur Regeneration der Epidermis und/oder zur Zellreparatur der Epidermis umfasst.

## Claims

1. A non-therapeutic use of at least one compound 6-O-(C8-C20 alkyl ester) of 1-O-(C1-C6 alkyl)-β-D-glucoside as an agent for regeneration of the epidermis and/or cellular repair of the epidermis.

2. The non-therapeutic use according to claim 1, **characterized in that** it is 6-O-lauroyl of 1-O-n-butyl-β-D-glucoside.

3. A dermatological composition, **characterized in that** it comprises as active ingredient at least one compound 6-O-(C8-C20 alkyl ester) of 1-O-(C1-C6 alkyl)-β-D-glucoside and at least one dermatologically acceptable excipient, for use thereof in the regeneration of the epidermis and/or cellular repair of the epidermis.

4. The dermatological composition for use thereof according to claim 3, **characterized in that** the amount of 6-0-(C8-C20 alkyl ester) of 1-O-(C1-C6 alkyl)-β-D-glucoside varies between 0.01 % and 10% by weight relative to the total weight of the composition.

5. The dermatological composition for use thereof according to one of claims 3 or 4, **characterized in that** the active ingredient is 6-O-lauroyl of 1-O-n-butyl-β-D-glucoside.

6. A non-therapeutic use of a cosmetic composition comprising as active ingredient at least one compound 6-O-(C8-C20 alkyl ester) of 1-O-(C1-C6 alkyl)-β-D-glucoside and at least one cosmetically acceptable excipient for regeneration of the epidermis and/or cellular repair of the epidermis.
